(19) Europäisches Patentamt
European Patent Office
Office européen des brevets

(11) **EP 3 231 874 A1**

(12) **EUROPEAN PATENT APPLICATION**

(43) Date of publication:
18.10.2017 Bulletin 2017/42

(51) Int Cl.:
*C12Q 1/68* (2006.01)

(21) Application number: 16165319.1

(22) Date of filing: 14.04.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Curetis GmbH
71088 Holzgerlingen (DE)

(72) Inventors:
• GALATA, Valentina
66123 Saarbrücken (DE)
• KELLER, Andreas
66346 Püttingen (DE)
• QUAST, Holger
10435 Berlin (DE)
• SCHMOLKE, Susanne
91052 Erlangen (DE)

(74) Representative: Schnappauf, Georg
ZSP Patentanwälte PartG mbB
Hansastraße 32
80686 München (DE)

Remarks:
The complete document including Reference Tables and the Sequence Listing can be downloaded from the EPO website

(54) **USING THE FULL REPERTOIRE OF GENETIC INFORMATION FROM BACTERIAL GENOMES AND PLASMIDS FOR IMPROVED GENETIC RESISTANCE TESTS**

(57) The invention relates to a method of determining an antimicrobial drug resistance profile for a microorganism, wherein nucleic acid sequences of the microorganism are analyzed for at least two genetic variations of the nucleic acid sequences comprising at least one genetic variation in a chromosome and at least one genetic variation in at least one plasmid, as well as a, e.g. diagnostic, method of determining an infection of a patient with a microorganism potentially resistant to antimicrobial drug treatment and a method of selecting a treatment of a patient suffering from an infection with a potentially resistant microorganism, wherein the data of the antimicrobial drug resistance profile are applied.

**Description**

The present invention relates to a method of determining an antimicrobial drug resistance profile for a microorganism, wherein nucleic acid sequences of the microorganism are analyzed for at least two genetic variations of the nucleic acid sequences comprising at least one genetic variation in a chromosome and at least one genetic variation in at least one plasmid, as well as a, e.g. diagnostic, method of determining an infection of a patient with a microorganism potentially resistant to antimicrobial drug treatment and a method of selecting a treatment of a patient suffering from an infection with a potentially resistant microorganism, wherein the data of the antimicrobial drug resistance profile are applied.

Antibiotic resistance is a form of drug resistance whereby a sub-population of a microorganism, e.g. a strain of a bacterial species, can survive and multiply despite exposure to an antibiotic drug. It is a serious health concern for the individual patient as well as a major public health issue. Timely treatment of a bacterial infection requires the analysis of clinical isolates obtained from patients with regard to antibiotic resistance, in order to select an efficacious therapy. Generally, for this purpose an association of the identified resistance with a certain microorganism (i.e. ID) is necessary.

Antibacterial drug resistance (ADR) represents a major health burden. The presence and genesis of bacterial resistance against active agents is more rapidly gaining importance as assumed. The increased usage of available drugs leads to multi-resistant bacteria, which in turn need even harder medical treatment. According to the World Health Organization's anti-microbial resistance global report on surveillance, ADR leads to 25,000 deaths per year in Europe and 23,000 deaths per year in the US. In Europe, 2.5 million extra hospital days lead to societal cost of 1.5 billion euro. In the US, the direct cost of 2 million illnesses leads to 20 billion dollar direct cost. The overall cost is estimated to be substantially higher, reducing the gross domestic product (GDP) by up to 1.6%.

In general the mechanisms for resistance of bacteria against antimicrobial treatments rely to a very substantial part on the organism's genetics. The respective genes or molecular mechanisms are either encoded in the genome of the bacteria or on plasmids that can be interchanged between different bacteria. The most common resistance mechanisms include:

  1) Efflux pumps are high-affinity reverse transport systems located in the membrane that transports the antibiotic out of the cell, e.g. resistance to tetracycline.
  2) Specific enzymes modify the antibiotic in a way that it loses its activity. In the case of streptomycin, the antibiotic is chemically modified so that it will no longer bind to the ribosome to block protein synthesis.
  3) An enzyme is produced that degrades the antibiotic, thereby inactivating it. For example, the penicillinases are a group of beta-lactamase enzymes that cleave the beta lactam ring of the penicillin molecule.

In addition, some pathogens show natural resistance against drugs. For example, an organism can lack a transport system for an antibiotic or the target of the antibiotic molecule is not present in the organism.

Pathogens that are in principle susceptible to drugs can become resistant by modification of existing genetic material (e.g. spontaneous mutations for antibiotic resistance, happening in a frequency of one in about 100 mio bacteria in an infection) or the acquisition of new genetic material from another source. One example is horizontal gene transfer, a process where genetic material contained in small packets of DNA can be transferred between individual bacteria of the same species or even between different species. Horizontal gene transfer may happen by transduction, transformation or conjugation. Usually, the expression of resistance imparting markers is induced only by presence of a drug.

Generally, testing for susceptibility/resistance to antimicrobial agents is performed by culturing organisms in different concentrations of these agents.

In brief, agar plates are inoculated with patient sample (e.g. urine, sputum, blood, stool) overnight. On the next day individual colonies are used for identification of organisms, either by culturing or using mass spectroscopy. Based on the identity of organisms new plates containing increasing concentration of drugs used for the treatment of these organisms are inoculated and grown for additional 12 - 24 hours. The lowest drug concentration which inhibits growth (minimal inhibitory concentration - MIC) is used to determine susceptibility/resistance for tested drugs. The process takes at least 2 to 3 working days during which the patient is treated empirically. Automated systems exist from several companies, e.g. Biomeriux (Vitek), Beckman Coulter (Microscan). A significant reduction of time-to-result is needed especially in patients with life-threatening disease and to overcome the widespread misuse of antibiotics.

More recent approaches focus on the genetic constitution of the pathogen and span the whole spectrum from low-plex testing for single resistance markers over small dedicated panels and target enriched sequencing to whole genome sequencing of bacteria. These approaches have demonstrated significant potential to revolutionize care of patients with infectious diseases.

Recent developments include PCR based test kits for fast bacterial identification (e.g. Biomerieux Biofire Tests, Curetis Unyvero Tests). With these test the detection of selected resistance loci is possible for a very limited number of drugs, but no correlation to culture based AST is given. Mass spectroscopy is increasingly used for identification of pathogens in clinical samples (e.g. Bruker Biotyper), and research is ongoing to establish methods for the detection of susceptibility/resistance against antibiotics.

The use of molecular techniques for direct detection of MRSA has become more commonplace especially for screening purposes. Resistance to methicillin is mediated via the mec operon which is part of the staphylococcal cassette chromosome mec (SCCmec). Recently PCR tests were introduced that are based on the detection of the right extremity sequence of the SCCmec in combination with S. aureus specific marker. Initial reports exist that describe culture based susceptibility reports despite detection of the presence of a resistance conferring gene.

It is known that drug resistance can be associated with genetic modifications such as polymorphisms or gene duplications/deletions. This holds for viruses, where resistance testing is established clinical practice (e.g. HIV genotyping). More recently, it has been shown that resistance has also genetic causes in bacteria and even higher organisms, such as humans where tumors resistance against certain cytostatic agents can be linked to genomic mutations.

Wozniak et al. (BMC Genomics 2012, 13(Suppl 7):S23) disclose genetic determinants of drug resistance in Staphylococcus aureus based on genotype and phenotype data. Stoesser et al. disclose prediction of antimicrobial susceptibilities for Escherichia coli and Klebsiella pneumoniae isolates using whole genomic sequence data (J Antimicrob Chemother 2013; 68: 2234-2244).

Chewapreecha et al (Chewapreecha et al (2014) Comprehensive Identification of single nucleotide polymorphisms associated with beta-lactam resistance within pneumococcal mosaic genes. PLoS Genet 10(8): e1004547) used a comparable approach to identify mutations in gram-positive Streptococcus Pneumonia.

However, there is a need for improved detection of infections with antimicrobial drug resistant microorganisms, particularly microbial species, and an improved prediction of response to anti-microbial therapy represents still a high unmet clinical need.

Summary of the invention

While other approaches of the state of the art focus usually on point mutations, earlier findings showed that improved performance of prediction of resistance can be gained in two ways:

  a) machine learning approaches can be used to combine the predictive power of single point mutations. These weak learners were located on the bacterial chromosome.

  b) for some drugs point mutations did not yield a high predictive power. Thus we added larger structural variants, such as genes that were present or absent to improve the classification accuracy.

Besides the chromosomes, genetic resistance information can be also encoded on plasmids of bacteria. These are e.g. exchanged between different strains using horizontal gene transfer. The inventors thus extended the previous analyses that were centered on chromosomal genes and first defined pan-genomes. These contain the full genetic set, from the chromosome and the plasmids. In a first pass point a) described above was reinforced. By using more genetic variants generally a better performance was reached.

As a result of the analysis, the inventors found out that a combination of detection of at least two genetic variations of the nucleic acid sequences comprising at least one genetic variation in a chromosome and at least one genetic variation in at least one plasmid can improve the diagnosis of resistant / susceptible microorganisms, particularly bacterial microorganisms, to antimicrobial, e.g. antibiotic, drugs.

According to a first aspect the present invention relates to a method of determining an antimicrobial drug resistance profile for a microorganism, comprising:

- obtaining or providing a first data set of nucleic acid sequences of a plurality of clinical isolates of the microorganism, wherein at least a part of the nucleic acid sequences of the first data set are assembled; and/or
  obtaining or providing a first data set of nucleic acid sequences of a plurality of clinical isolates of the microorganism and aligning the nucleic acid sequences of the first data set to at least one reference sequence;
- analyzing the nucleic acid sequences of the first data set for at least two genetic variations of the nucleic acid sequences comprising at least one genetic variation in a chromosome and at least one genetic variation in at least one plasmid to obtain a third data set of structural variants;
- providing a second data set of antimicrobial drug, e.g. antibiotic, resistance and/or susceptibility of the plurality of clinical isolates of the microorganism;
- correlating the third data set with the second data set and statistically analyzing the correlation; and
- determining the genetic variations in the nucleic acid sequences of the microorganism associated with antimicrobial drug, e.g. antibiotic, resistance.

Furthermore discloses is - in a second aspect - a, e.g. diagnostic, method of determining an infection of a patient with an antimicrobial drug resistant microorganism, comprising the steps of:

> a) obtaining or providing a sample containing or suspected of containing a microorganism from the patient;
> b) determining the presence of at least two genetic variations of the nucleic acid sequences comprising at least one genetic variation in the chromosome and at least one genetic variation in at least one plasmid, as determined by the method of the first aspect, wherein the presence of said at least two genetic variations of the nucleic acid sequences comprising at least one genetic variation in the chromosome and at least one genetic variation in at least one plasmid is indicative of an infection with an antimicrobial drug resistant microorganism in said patient.

In addition, a method of selecting a treatment of a patient suffering from an infection with a potentially antimicrobial drug resistant microorganism is disclosed in a third aspect, comprising the steps of:

> a) obtaining or providing a sample containing or suspected of containing a microorganism from the patient;
> b) determining the presence of at least two genetic variations of the nucleic acid sequences comprising at least one genetic variation in the chromosome and at least one genetic variation in at least one plasmid, as determined by the method of the first aspect, wherein the presence of said at least two genetic variations of the nucleic acid sequences comprising at least one genetic variation in the chromosome and at least one genetic variation in at least one plasmid is indicative of a resistance to one or more antimicrobial drugs;
> c) identifying said at least one or more antimicrobial drugs; and
> d) selecting one or more antimicrobial drugs different from the ones identified in step c) and being suitable for the treatment of the infection with the microorganism.

In a further aspect the present invention is directed to a computer program product comprising computer executable instructions which, when executed, perform a method according to either of the first, second and third aspect.

Even further aspects and embodiments of the invention are disclosed in the dependent claims and can be taken from the following description and examples, without being limited thereto.

Detailed description of the invention

Definitions

Unless defined otherwise, technical and scientific terms used herein have the same meaning as commonly understood by one of ordinary skill in the art to which this invention belongs.

Susceptibility herein means that isolates are inhibited by a certain concentration of an antimicrobial agent, whereas resistance means that isolates are not inhibited

An "antimicrobial drug" in the present invention refers to a group of drugs that includes antibiotics, antifungals, antiprotozoals, and antivirals. According to certain embodiments, the antimicrobial drug is an antibiotic.

The term "nucleic acid molecule" refers to a macromolecule comprising nucleotides, particularly a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA.

The term "nucleic acid sequence information" relates to information which can be derived from the sequence of a nucleic acid molecule, i.e. the nucleic acid sequence, such as the sequence itself or a variation in the sequence as compared to a reference sequence. A genetic sequence can thereby encompass coding as well as non-coding parts. The whole genetic material of a microorganism thereby makes up the genome.

The term "genetic variation", which also can be termed "mutation", relates to a variation in the sequence as compared to a reference sequence or multiple reference sequences. Such a reference sequence can be e.g. determined in a predominant wild type organism or another reference organism, e.g. a defined and known bacterial strain or substrain. A mutation is for example a deletion of one or multiple nucleotides, an insertion of one or multiple nucleotides, or substitution of one or multiple nucleotides, duplication of one or a sequence of multiple nucleotides, translocation of one or a sequence of multiple nucleotides, e.g. also a single nucleotide polymorphism (SNP). The term "single nucleotide polymorphism" (SNP) is thereby synonymous to the term "single nucleotide variant" (SNV), and both refer to the same.

In the context of the present invention a "sample" is a sample which comprises at least one nucleic acid molecule from a bacterial microorganism. Examples for samples are: cells, tissue, biopsy specimens, body fluids such as blood, urine, saliva, sputum, plasma, serum, cell culture supernatant, swab sample and others. According to certain embodiments, the sample is a patient sample (clinical isolate).

New and highly efficient methods of sequencing nucleic acids referred to as next generation sequencing have opened the possibility of large scale genomic analysis. The term "next generation sequencing" or "high throughput sequencing" refers to methods achieving a higher throughput in sequencing, e.g. high-throughput sequencing technologies that parallelize the sequencing process, producing thousands or millions of sequences at once, or methods producing longer reads and are read out faster. Examples include Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope(TM) single molecule sequencing, Single Molecule SMRT(TM) sequencing, Single Molecule real time (RNAP) sequencing, Nanopore DNA sequencing, Sequencing By Hybridization, Amplicon Sequencing, GnuBio.

Within the present description the term "microorganism" comprises the term microbe. The type of microorganism is not particularly restricted, unless noted otherwise or obvious, and, for example, comprises bacteria, viruses, fungi, microscopic algae und protozoa, as well as combinations thereof. According to certain aspects, it refers to one or more bacterial species, being either Gram-negative or Gram-positive, e.g. one or more of Acinetobacter, e.g. Acinetobacter baumannii, Escherichia, e.g. E.coli, Enterobacter, Klebsiella, e.g. Klebsiella oxytoca and/or Klebsiella pneumoniae, Proteus, e.g. Proteus mirabilis, Pseudomonas, Salmonella, Serratia, e.g. Serratia marcescens, Shigella and/or Staphylococcus species.

A reference to a microorganism or microorganisms in the present description comprises a reference to one microorganism as well a plurality of microorganisms, e.g. two, three, four, five, six or more microorganisms.

A vertebrate within the present invention refers to animals having a vertebrae, which includes mammals - including humans, birds, reptiles, amphibians and fishes. The present invention thus is not only suitable for human medicine, but also for veterinary medicine.

According to certain embodiments, the patient in the present methods is a vertebrate, more preferably a mammal and most preferred a human patient.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

Regarding the dosage of the antimicrobial, e.g. antibiotic, drugs, it is referred to the established principles of pharmacology in human and veterinary medicine. For example, Forth, Henschler, Rummel "Allgemeine und spezielle Pharmakologie und Toxikologie", 9th edition, 2005, pp. 781-919 might be used as a guideline. Regarding the formulation of a ready-to-use medicament, reference is made to "Remington, The Science and Practice of Pharmacy", 22nd edition, 2013, pp.777-1070.

Assembling of a nucleic acid, e.g. gene, sequence can be carried out by any known method and is not particularly limited.

According to certain embodiments, mutations, respectively genetic variations, that were found using alignments can also be compared or matched with alignment-free methods, e.g. for detecting single base exchanges, for example based on contigs that were found by assemblies. For example, reads obtained from sequencing can be assembled to contigs and the contigs can be compared to each other.

In the description, the term "structural variations" is used equivalently to the term "structural changes", and both refer to the same phenomenon within the scope of this invention.

A structural variation comprising a change in the nucleic acid sequence comprising more than one base refers to a structural variation wherein at least two bases, preferably at least four bases, in a nucleic acid sequence of a microorganism that are adjacent are changed, and can refer to e.g. a deletion of multiple (2, e.g. 4, or more) nucleotides, an insertion of multiple (2, e.g. 4, or more) nucleotides, a substitution of multiple (2, e.g. 4, or more) nucleotides, a duplication of a sequence of multiple (2, e.g. 4, or more) nucleotides, or a translocation of a sequence of multiple (2, e.g. 4, or more) nucleotides. According to certain embodiments, a structural variation affects a sequence length of at least about 50 bases, preferably at least about 100 bases, further preferably at least about 1 Kb (= 1000 bases). According to certain embodiments, a structural variation affects a sequence length of at most 300 Mb (Mega base = 1000000 bases), e.g. of at most 30 Mb, e.g. of at most 3Mb. In case the term "structural variation refers to a change in the nucleic acid sequence of 4 or more bases, e.g. at least about 50 bases, preferably at least about 100 bases, further preferably at least about 1 Kb, the term single nucleotide polymorphism can be understood to include also small indels (insertions or deletions) of up to at most 3 bases, e.g. up to two bases. According to certain embodiments, a structural variation can comprise bigger parts sections of the nucleic acid sequence, e.g. at least one whole gene in the nucleic acid sequence of the microorganism, or even more genes in an open reading frame. According to certain embodiments, structural variations refer to inclusion of repetitive elements, copy number variations (gains and losses of single genes or larger parts of chromosomes), gene fusions, translocations and other more rare events. According to certain embodiments, at least one inclusion of repetitive elements, one copy number variation (gains and losses of single genes or larger parts of chromosomes), one gene fusion, and/or translocation of single genes or larger parts of chromosomes is observed in the present methods as a structural variation. Structural variations can e.g. include inclusion of repetitive elements, copy number variations (gains and losses of single genes or larger parts of chromosomes), gene fusions, translocations, inclusion/addition of new genes, and other more rare events.

A single nucleotide polymorphism (SNP) refers within the scope of the invention to a variation in a single nucleotide within a nucleic acid sequence, which can result from e.g. an addition, deletion, substitution, insertion or translocation of a single nucleotide.

In the present invention, a reference sequence is not particularly limited, as long as it is useful as a reference for one or more unknown nucleic acid sequences in one or more samples. It can, for example, be one or more reference nucleic acid sequences, e.g. reference genomes, a pan-genome or one or more centroids. A pan-genome, also referred to as supra-genome, can describe the full complement of genes in a clade, e.g. a certain species in bacteria, which can vary among related strains. According to certain embodiments, the reference sequences comprise one or more centroids, wherein a centroid is a representative of a gene group/family/cluster of a genome, e.g. of a microorganism. Centroids can be for example extracted from the database MetaRef (http://metaref.org/). After the extraction the data from the MetaRef database can be updated continually for further experiments. A list of centroids can be extracted for each organism separately or as a whole. The centroid information, e.g. for annotation, can be extracted from databases like IMG (http://img.jgi.doe.gov/), as in the present case, or NCBI. According to certain embodiments, alignment is carried out using a pan-genome.

According to a first aspect, the present invention relates to a method of determining an antimicrobial drug resistance profile for a microorganism, comprising:

- obtaining or providing a first data set of nucleic acid sequences of a plurality of clinical isolates of the microorganism, wherein at least a part of the nucleic acid sequences of the first data set are assembled; and/or
obtaining or providing a first data set of nucleic acid sequences of a plurality of clinical isolates of the microorganism and aligning the nucleic acid sequences of the first data set to at least one reference sequence;
- analyzing the nucleic acid sequences of the first data set for at least two genetic variations of the nucleic acid sequences comprising at least one genetic variation in a chromosome and at least one genetic variation in at least one plasmid to obtain a third data set of structural variants;
- providing a second data set of antimicrobial drug, e.g. antibiotic, resistance and/or susceptibility of the plurality of clinical isolates of the microorganism;
- correlating the third data set with the second data set and statistically analyzing the correlation; and
- determining the genetic variations in the nucleic acid sequences of the microorganism associated with antimicrobial drug, e.g. antibiotic, resistance.

In this method, as well as the other methods of the invention, the first data set of nucleic acid, e.g. gene, but also non-coding, sequences of a plurality of clinical isolates can be provided or obtained in any way, preferably non-invasive, and can be e.g. provided from *in vitro* samples.

In the present methods, at least two genetic variations of the nucleic acid sequences comprising at least one genetic variation in a chromosome and at least one genetic variation in at least one plasmid are determined, leading to improved results compared to determining at least two genetic variations in only chromosomal nucleic acid sequences or only in plasmid nucleic acid sequences.

In the present methods, at least two genetic variations of the nucleic acid sequences comprising at least one genetic variation in a chromosome and at least one genetic variation in at least one plasmid are determined, i.e. at least a genetic variation in a chromosome of the microorganism and at least a genetic variation in at least one plasmid. A plasmid is thereby a small nucleic acid molecule within a cell, e.g. in a microorganism, that is physically separated from chromosomal nucleic acid. According to certain aspects, more than one, e.g. two, three, four, five, six, seven, eight, nine, ten, eleven, twelve, or more, genetic variations in a chromosome and/or more than more than one, e.g. two, three, four, five, six, seven, eight, nine, ten, eleven, twelve, or more, genetic variation in at least one plasmid are determined. The genetic variation(s) in the plasmid can be determined in one plasmid or more than one plasmid, e.g. two, three, four, five, six, seven, eight, nine, ten, or more plasmids. According to certain embodiments, the correlation and statistical analysis can even encompass a technique wherein all genetic variations in the chromosomal and plasmid nucleic acid sequences are taken into account and then optimized for obtaining the genetic variations in the nucleic acid sequences of the microorganism associated with antimicrobial drug, e.g. antibiotic, resistance which have improved statistical relevance, e.g. can obtain a higher probability to be association with antimicrobial drug, e.g. antibiotic, resistance. According to certain embodiments, a statistical analysis can be carried out using a classification approach/method like a decision tree, random forest, neural network, bayesian classification, support vector machine, etc. wherein at first the presence of a single nucleotide polymorphism and/or structural variation is determined, e.g. a decision tree, wherein in the decision tree at first the presence of a single nucleotide polymorphism and/or structural variation is determined. A classification approach can be suitably selected and applied, e.g. a decision tree can be generated using known methods, e.g. within the scope of the statistical analysis, and is otherwise not particularly restricted. According to certain embodiments, a resistance in the microorganism can be determined using a decision tree, corresponding to a statistical analysis.

According to certain embodiments, the obtaining or providing of nucleic acid, e.g. gene, but also e.g. non-coding, sequences of a plurality of clinical isolates in this method - as well as the other methods of the invention - can comprise the following:

A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of at least one microorganism of interest, particularly a bacterial microorganism. For example, sequencing can be carried out using polymerase chain reaction (PCR), particularly multiplex PCR, or

high throughput sequencing or next generation sequencing, preferably using high-throughput sequencing. For sequencing, preferably an *in vitro* sample is used.

The obtaining or providing of nucleic acid sequences thereby included obtaining or providing chromosomal nucleic acid sequences as well as plasmid nucleic acid sequences, particularly the whole nucleic acid sequences of the microorganism, e.g. bacterial microorganism.

The data obtained by the sequencing can be in any format, and can then be used to identify the nucleic acids of the microorganism to be identified, by known methods, e.g. fingerprinting methods, comparing nucleic acid sequences, e.g. genomes and/or aligning to at least one, or more, reference sequences (chromosomal and plasmid sequences) of one or more species of the microorganism of interest, e.g. a reference genome and/or centroids, etc., forming a third data set of, optionally aligned, nucleic acid sequences, e.g. genes, for a microorganism - discarding additional data from other sources, e.g. the vertebrate. For the present method, also the raw data can be used and/or assemblies, at least in part, can be used for forming the third data set. Thus, according to certain embodiments, at least a part of the nucleic acid, e.g. gene, sequences of the first data set can be assembled, wherein assembly can be carried out by any known method and is not particularly limited. In addition, also data from reference sequences, e.g. centroids and/or genomes of known species, e.g. from bacterial species that are already known, e.g. using databases like MetaRef - which can provide pan-genomes - and/or at the NCBI, can be used in the first data set and/or for evaluation of the first data set. For constructing pan-genomes, also assembled data can be used, e.g. nucleic acid sequence data obtained by sequencing of samples can be assembled and then calculated, e.g. using Roary (Rapid large-scale prokaryote pan genome analysis (Bioinformatics 2015 Nov 15;31(22): 3691-3. doi:

10.1093/bioinformatics/btv421. Epub 2015 Jul 20. Page AJ, Cummins CA, et al.).

Pan-genomes can thereby offer the advantage that they contain chromosomal nucleic acid sequences as well as plasmid nucleic acid sequences, i.e. a comparison to the pan-genome enables a fast and complete analysis of the first data set for genetic variations. Further, a pan-genome also allows for a more complete analysis for genetic variations as the pan-genome also allows for variation in gene content among closely related strains.

For some organisms, it might be useful in genome-wide association studies to reference the points of interest, e.g. structural variations and/or SNPs, to one constant reference for enhanced standardization. In case of the human with a high consistency of the genome and 99% identical sequences among individuals this is easy and represents the standard, as corresponding reference genomes are available in databases.

In case of organisms that trigger infectious diseases (e.g. bacteria and viruses) this is much more difficult, though, and particularly also genetic variations like structural variations and/or SNPs that are not on genes, particularly known genes, can be missed when aligning sequence data to a reference nucleic acid sequence, e.g. a reference genome. One possibility to overcome this is to fall back on a virtual pan-genome which contains all sequences of a certain genus or to perform reference free variation calling. A further possibility is the analysis of a huge amount of reference sequences, e.g. using MetaRef, and even all available references, which is much more complex. Therein all n references from a database (e.g. RefSeq) are extracted and compared with the newly sequenced bacterial nucleic acid sequences, e.g. genomes, $k$. After this, matrices (% of mapped reads, % of covered nucleic acid sequence, e.g. genome) can be applied and the data can be compared to several reference sequences. In such a case, $n \times k$ complete alignments are carried out. Having a big number of references, stable results can be obtained.

In the present method, nucleic acid, e.g. gene, but also non-coding, sequence of the first data set can also be assembled, at least in part, according to certain embodiments with known methods, e.g. by de-novo assembly or mapping assembly, reference guided assembly. The sequence assembly is not particularly limited, and any known nucleic acid sequence assembler can be used, e.g. based on Sanger, 454, Solexa, Illumina, SOLid technologies, etc., as well as hybrids/mixtures thereof.

According to certain embodiments, the data of nucleic acids of different origin than the microorganism of interest, e.g. a bacterial microorganism, can be removed after the nucleic acids of interest are identified, e.g. by filtering the data out. Such data can e.g. include nucleic acids of a patient, e.g. the vertebrate, e.g. human, and/or other microorganisms, etc. This can be done by e.g. computational subtraction, as developed by Meyerson et al. 2002. For this, also aligning to the nucleic acid sequences, e.g. genome, of the vertebrate, etc., is possible. For aligning, several alignment-tools are available. This way the original data amount from the sample can be drastically reduced.

After such removal of "excess" data, obtaining the third data set can be carried out for the microorganism, e.g. a bacterial microorganism, as described above and below.

Using these techniques, structural variations and SNPs in the nucleic acid sequences, e.g. in the gene sequences, of the microorganism of interest, e.g. a bacterial microorganism, can be obtained for various species, including chromosomal nucleic acid sequences as well as plasmid nucleic acid sequences.

When testing these same species for antimicrobial drug, e.g. antibiotic, susceptibility of a number of antimicrobial drugs, e.g. antibiotics, e.g. using standard culturing methods on dishes with antimicrobial drug, e.g. antibiotic, intake, as e.g. described below, the results of these antimicrobial drug, e.g. antibiotic, susceptibility tests can then be cross-referenced/correlated with the structural variations in the nucleic acid sequences of the respective microorganism. Using several, e.g. 50 or more than 50, 100 or more than 100, 200 or more than 200, 400 or more than 400, 500 or more than 500, 800 or more than 800, 900 or more than 900, 1000 or more than 1000, or 1100 or more than 1100 different isolates of the same or different species of a microorganism, statistical analysis can be carried out on the obtained cross-referenced data between genetic variations and antimicrobial drug, e.g. antibiotic, susceptibility for these microorganisms, using known methods.

Regarding culturing methods, which are nor limited, samples of microorganisms can be e.g. cultured overnight. On the next day individual colonies can be used for identification of organisms, either by culturing or using mass spectroscopy. Based on the identity of organisms new plates containing increasing concentration of antibiotics used for the treatment of these organisms are inoculated and grown for additional 12 - 24 hours. The lowest drug concentration which inhibits growth (minimal inhibitory concentration - MIC) can be used to determine susceptibility/resistance for tested antibiotics.

Also, resistance testing can be carried out by determining e.g. known resistance genes in the different isolates, like in case of methicillin resistant Staphylococcus aureus (MRSA) and methicillin susceptible Staphylococcus aureus (MSSA). For determining resistances, respectively susceptibility, the data from culturing methods and/or from determining known resistance genes, as well as data obtained in different ways, e.g. based on mass spectrometry (possibly also in connection with culturing) can be used.

Correlation of the genetic variations with antimicrobial drug, e.g. antibiotic, resistance can be carried out in a usual way and is not particularly limited. For example, resistances can be correlated to structural variations and/or SNPs in the whole nucleic acid sequence(s) of the respective microorganism or only parts thereof, for example only coding parts of the nucleic acid sequence(s). In some cases even only genetic variations, i.e. structural variations and/or SNPS in nucleic acid molecules with certain nucleic acid sequences, e.g. genes, e.g. certain genes, or certain mutations in nucleic acid molecules with certain nucleic acid sequences, e.g. genes, can be determined. After correlation, statistical analysis can be carried out. The correlation is carried out with both chromosomal nucleic acid sequences and plasmid nucleic acid sequences.

According to certain embodiments, the data of the first data set, particularly genetic variations, particularly structural variations and/or SNPs, can be filtered prior to a possible annotation to a pan-genome and/or reference genome(s) and the correlation with the resistance/susceptibility data, e.g. when determining structural variations.

For example, to reduce the number of similar annotations for genetic variations, e.g. for structural variations and/or SNPs, they can be filtered and aggregated by one or more of the following:

- Only annotations for which the considered genetic variation, e.g. structural variation and/or SNP, lies on a protein can be kept and the further data discarded
- Only annotations which do not contain "hypothetical proteins" can be kept
- Annotations can be sorted by identification number (ID) for genetic variations, e.g. for SNPs and/or structural variation, and nucleic acid sequence, e.g. gene product
- For a unique pair of IDs and nucleic acid sequences, e.g. gene products, only the first annotation can be kept, e.g. in case of multiple nucleic acid sequences, e.g. coding certain genes, in a genome

Also, according to certain embodiments, the following genetic variations, e.g. structural variations and/or SNPs, can be excluded:

1. Constant features and phenotypes (same value or only NA (not applicable)) can be removed (e.g. centroids present in all samples or phenotypes with the result "resistant" for all samples)
2. Almost constant features and phenotypes can also be removed, e.g. features whose most frequent value was in >=95% of all samples, ignoring NA values, can be removed (e.g. a centroid is present in >=95% of all samples)
∘ Also phenotypes whose most frequent value was in >=90% of all samples, ignoring NA values, can be removed (e.g. >=90% of all samples are resistant)
3. In addition, only drugs with non-missing data for at least 10% of the samples can be kept.
4. Genetic variations, e.g. SNPs and/or structural variations, without any annotation or mutations, e.g. SNPs and/or structural variations whose all annotations contain flag "synonymous", can be removed so that only mutations, e.g. SNPs and/or structural variations, with at least one non-synonymous annotation, e.g. a non-synonymous coding, are considered

Statistical analysis is not particularly limited and can be suitably carried out. Statistical analysis of the correlation of the genetic, e.g. gene, variations with antimicrobial drug, e.g. antibiotic, resistance is not particularly limited and can be carried out, depending on e.g. the amount of data, in different ways, for example using analysis of variance (ANOVA), Student's t-test or Fisher's exact test, for example with a sample size n of 50, 100, 200, 300, 400, 500, 600, 800, 1000 or 1100, and a level of significance ($\alpha$-error-level) of e.g. 0.05 or smaller, e.g. 0.05, preferably 0.01 or smaller. According to certain embodiments, statistical analysis in the present methods can be carried out using Fisher's test - or a similar test - with $p < 10^{-3}$, preferably $p < 10^{-6}$, further preferably $p < 10^{-9}$. A statistical value can be obtained for each genetic variation, e.g. structural variation and/or SNP, for each nucleic acid / genetic sequence in the nucleic acid sequences, i.e. the chromosomal and plasmid nucleic acid sequences, as well as for all antibiotics tested, a group of antibiotics or a single antibiotic. The obtained p-values can also be adapted for statistical errors, if needed.

For statistically sound results a multitude of individuals should be sampled, with n = 50, 100, 200, 300, 400, 500, 600, 800, 1000, or 1100 or more and a level of significance ($\alpha$-error-level) of e.g. 0.05 or smaller, e.g. 0.05, preferably 0.01 or smaller. According to certain embodiments, particularly significant results can be obtained for n = 200, 300, 400, 500, 600 or more.

For statistically sound results a multitude of individuals should be sampled, with n = 50 or more, 100 or more, 200 or more, 300 or more, 400 or more, 500 or more, 600 or more, 800 or more, 1000 or more, or 1100 or more, and a level of significance ($\alpha$-error-level) of e.g. 0.05 or smaller, e.g. 0.05, preferably 0.01 or smaller. According to certain embodiments, particularly significant results can be obtained for n = 200 or more, 300 or more, 400 or more, 500 or more, 600 or more, 800 or more, 1000 or more, or 1100 or more.

For statistical analysis, e.g. Fisher's exact two-sided test can be applied with subsequent p-value adjustment over all phenotypes together using e.g. familywise error rate (FWER) or FDR (false discovery rate) and p-value threshold of 0.01 (corresponding to $10^{-2}$, respectively 1e-2). Additionally, 10 permutation tests can be performed by permuting each phenotype separately and applying Fisher's exact test, e.g. to the centroid presence matrix and permuted phenotypes. Regarding centroids, the results then can be further filtered by centroid annotation, i.e.

1. Centroids without a gene product name can optionally be removed
2. Centroids whose gene product name contains "putative", "predicted" or "hypothetical" can be removed
3. If there are centroids with same gene product name and gene symbol than only the first one can be kept
4. Centroids without GeneBank accession can be removed

Other statistical analysis can also be carried out alternatively or in addition, though.
According to certain embodiments, the genetic variations, e.g. structural variations and/or SNPs can be annotated to a pan-genome of the microorganism and/or annotated to one or more reference sequences, e.g. centroids, of the microorganism. The construction of a pan-genome is not particularly limited and can be done using known methods. For example, assembled data, e.g. of several, e.g. more than 100, more than 200, or more than 300, individual samples of the microorganism, can be used for constructing a pan-genome, e.g. nucleic acid sequence data obtained by sequencing of the samples can be assembled and then calculated, e.g. using Roary (Rapid large-scale prokaryote pan genome analysis (Bioinformatics 2015 Nov 15;31(22): 3691-3. doi:

10.1093/bioinformatics/btv421. Epub 2015 Jul 20. Page AJ, Cummins CA, et al.). According to certain embodiments, the genetic variations, e.g. structural variations and/or SNPs can be annotated to a pan-genome of the microorganism. However, other suitable reference genomes can be found at publicly available data bases like at the NCBI or from MetaRef.

When referring to the second data set, wherein the second data set e.g. comprises, respectively is, a set of antimicrobial drug, e.g. antibiotic, resistances of a plurality of clinical isolates, this can, within the scope of the invention, also refer to a self-learning data base that, whenever a new sample is analyzed, can take this sample into the second data set and thus expand its data base. The second data set thus does not have to be static and can be expanded, either by external input or by incorporating new data due to self-learning. This is, however, not restricted to the first aspect of the invention, but applies to other aspects of the invention that refer to a second data set, which does not necessarily have to refer to antimicrobial drug resistance. The same applies, where applicable, to the first data set, e.g. in the first aspect.

According to certain embodiments of the first aspect, the genetic variations, e.g. SNPs and/or structural variations, are detected alignment-free. According to certain embodiments, the genetic variations, e.g. SNPs and/or structural variations, are annotated to a pan-genome of the microorganism and/or annotated to one or more reference sequences.

The method of the first aspect of the present invention, as well as related methods, e.g. according to the 2nd and 3rd aspect, comprise correlating different genetic variations to each other. This way higher statistical significance can be achieved. Particularly, improved results are obtained by correlating genetic variations in chromosomal nucleic acid sequences and plasmid nucleic acid sequences.

According to certain embodiments of the method of the first aspect and related methods - as above, the second data set can be provided by culturing the clinical isolates of the microorganism on suitable plates, e.g. agar plates, provided with antimicrobial drugs, e.g. antibiotics, at different concentrations, and the second data can be obtained by taking the minimal concentration of the plates that inhibits growth of the respective microorganism.

According to certain embodiments the antimicrobial drug, e.g. antibiotic drug, is selected from the group consisting of β-lactams, β-lactam inhibitors, quinolones and derivatives thereof, e.g. fluoroquinolones, aminoglycosides, glycopeptides, lincosamides, macrolides, nitrofuranes, oxazolidinones, polyketides, respectively tetracyclines, and folate synthesis inhibitors, e.g. benzene derived/sulfonamide antibiotics. According to certain embodiments, the antimicrobial drug, e.g. antibiotic drug, is selected from the group consisting of Amoxicillin/K Clavulanate (AUG), Ampicillin (AM), Aztreonam (AZT), Cefazolin (CFZ), Cefepime (CPE), Cefotaxime (CFT), Ceftazidime (CAZ), Ceftriaxone (CAX), Cefuroxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T), Ampicillin/Sulbactam (A/S), Tetracycline (TE), Tobramycin (TO), and Trimethoprim/Sulfamethoxazole (T/S). According to certain embodiments, the microorganism is a Gram-positive or a Gram-negative bacteria, e.g. a Gram-negative bacteria.

In the methods of the invention, the resistance of the microorganism, particularly the bacterial microorganism, to one or more antimicrobial, e.g. antibiotic, drugs can be determined.

According to certain embodiments, the resistance of a microorganism, particularly bacterial microorganism, against 1, 2, 3, 4, 5, 6, 7, 8, 9, 10, 11, 12, 13, 14, 15 or 16, 17, 18, 19, 20, 21 or more antibiotic drugs is determined. According to certain embodiments, the resistance of a microorganism, particularly bacterial microorganism, against 1, 2, 3, 4, 5, 6, 7, 8, 9, 10, 11, 12, 13, 14, 15 or 16, 17, 18, 19, 20 or 21 antibiotic drugs is determined.

A second aspect of the present invention relates to a, e.g. diagnostic, method of determining an infection of a patient with an antimicrobial drug resistant microorganism, comprising the steps of:

a) obtaining or providing a sample containing or suspected of containing a microorganism from the patient;
b) determining the presence of at least two genetic variations of the nucleic acid sequences comprising at least one genetic variation in the chromosome and at least one genetic variation in at least one plasmid, as determined by the method of the first aspect, wherein the presence of said at least two genetic variations of the nucleic acid sequences comprising at least one genetic variation in the chromosome and at least one genetic variation in at least one plasmid is indicative of an infection with an antimicrobial drug resistant microorganism in said patient.

According to certain embodiments, the microorganism in step a) is a bacterial microorganism potentially resistant to antimicrobial drug treatment.

An infection of a patient with a microorganism, preferably a bacterial microorganism, e.g. one or more of Acinetobacter, Escherichia, e.g. E.coli, Enterobacter, Klebsiella, Proteus, Pseudomonas, Salmonella, Serratia, Shigella and/or Staphylococcus species, potentially resistant to antimicrobial drug treatment herein means an infection of a patient with a microorganism, preferably a bacterial microorganism, particularly one as noted above, wherein it is unclear if the microorganism, preferably bacterial microorganism, is susceptible to treatment with a specific antimicrobial drug or if it is resistant to the antimicrobial drug.

With this method, any genetic variations / mutations in the nucleic acid sequences of a microorganism, e.g. bacterial microorganism, e.g. a clinical isolate with an unknown strain of the microorganism, particularly bacterial microorganism, correlated with antimicrobial drug, e.g. antibiotic, resistance can be determined and a thorough antimicrobial drug, e.g. antibiotic, resistance profile can be established comprising structural variations as well as SNPs.

Again, the different steps can herein be carried out as described with regard to the first aspect of the present invention.

According to this aspect, an infection with a microorganism, particularly a bacterial microorganism, in a patient can be determined using sequencing methods of chromosomal and plasmid nucleic acid sequences, as well as a resistance to anti-microbial drugs, e.g. antibiotics, of the microorganism can be determined in a short amount of time compared to conventional methods, and a more thorough diagnostic is possible compared to a determination of only structural variations and/or SNPs in chromosomal nucleic acid sequences or only in plasmid nucleic acid sequences, leading to improved results for determining the resistance and/or susceptibility of the microorganism, particularly bacterial microorganism.

In a third aspect, the present invention relates to a method of selecting a treatment of a patient suffering from an infection with a potentially antimicrobial drug resistant microorganism, comprising the steps of:

a) obtaining or providing a sample containing or suspected of containing a microorganism from the patient;
b) determining the presence of at least two genetic variations of the nucleic acid sequences comprising at least one genetic variation in the chromosome and at least one genetic variation in at least one plasmid, as determined by the method of the first aspect, wherein the presence of said at least two genetic variations of the nucleic acid sequences comprising at least one genetic variation in the chromosome and at least one genetic variation in at least one plasmid is indicative of a resistance to one or more antimicrobial drugs;
c) identifying said at least one or more antimicrobial drugs; and
d) selecting one or more antimicrobial drugs different from the ones identified in step c) and being suitable for the treatment of the infection with the microorganism.

This method can be carried out similarly to the one in the second aspect of the invention and enables a fast way to select a suitable treatment with antibiotics for any infection with an unknown microorganism, particularly bacterial microorganism, with improved results compared to a determination of only structural variations and/or SNPs in chromosomal nucleic acid sequences or only in plasmid nucleic acid sequences.

In this method, as well as similar ones, no aligning is necessary, as the unknown sample can be directly correlated, after the nucleic acid sequences are produced, with the second data set, and thus genetic variations and antimicrobial drug, e.g. antibiotic, resistances can be determined. The first data set can be assembled, for example, using known techniques.

According to certain embodiments, statistical analysis in the present method is carried out using Fisher's test with $p < 10^{-3}$, preferably $p < 10^{-6}$, preferably $p < 10^{-9}$. Also, according to certain embodiments, the method further comprises correlating different genetic sites to each other.

According to certain aspects, genetic variations, e.g. structural variations and/or SNPs, in at least two, three, four, five, six, seven, eight, nine, ten, eleven, twelve, or more positions, respectively sequences, are determined in any of the methods of the present invention, e.g. in at least two positions, respectively sequences, or in at least three positions, respectively sequences, in the chromosomal nucleic acid sequences and/or the plasmid nucleic acid sequences. Instead of testing only single positions and/or sequences, the combination of several genetic variations, e.g. variant positions and/or sequences, can improve the prediction accuracy and further reduce false positive findings that are influenced by other factors. Therefore, it is in particular preferred to determine the presence of structural variations and/or SNPs in 2, 3, 4, 5, 6, 7, 8, 9, 10, 11, 12 (or more) sequences.

The identification of the at least one or more antimicrobial, e.g. antibiotic, drug in step c) is then based on the results obtained in step b) and corresponds to the antimicrobial, e.g. antibiotic, drug(s) that correlate(s) with the structural variations and SNPs. Once these antimicrobial drugs, e.g. antibiotics, are ruled out, the remaining antimicrobial drugs, e.g. antibiotic drugs/antibiotics, can be selected in step d) as being suitable for treatment.

According to certain embodiments in the second or third aspect, step b) is carried out using a classification approach/method like a decision tree, random forest, neural network, bayesian classification, support vector machine, etc. wherein at first the presence of a single nucleotide polymorphism and/or structural variation is determined, e.g. a decision tree, wherein in the decision tree at first the presence of a single nucleotide polymorphism and/or structural variation is determined. A classification approach can be suitably selected and applied, e.g. a decision tree can be generated using known methods, e.g. within the scope of the statistical analysis, and is otherwise not particularly restricted. According to certain embodiments, a resistance in the microorganism can be determined using a decision tree, corresponding to a statistical analysis. This way the diagnosis of a resistant microorganism, e.g. bacterial microorganism, can be optimized.

According to certain embodiments, determining the nucleic acid sequence information or the presence of a genetic variation in the present methods comprises using a next generation sequencing or high throughput sequencing method, e.g. as mentioned above.

According to certain embodiments in any of the aspects of the present invention, the microorganism is of genus Acinetobacter, particularly *Acinetobacter baumannii,* and the drug is chosen from CP, IMP, and/or LVX, and/or the microorganism is of genus Escherichia, particularly *E. coli,* and the drug is CRM, and/or the microorganism is of genus Klebsiella, particularly *Klebsiella oxytoca,* and the drug is CP, and/or the microorganism is of genus Klebsiella, particularly *Klebsiella pneumoniae,* and the drug is chosen from A/S, AZT, CAZ, CRM, and/or GM, and/or the microorganism is of genus Proteus, particularly *Proteus mirabilis,* and the drug is chosen from AM, A/S, CP, LVX, and/or T/S, and/or the microorganism is of genus Serratia, particularly *Serratia marcescens,* and the drug is chosen from AZT, CAX, CAZ, and/or CFT.

According to certain embodiments of any of the methods of the present invention the genetic variation is selected from at least one of structural variations of the nucleic acid sequences comprising at least a change in the nucleic acid sequence comprising more than one base, and single nucleotide polymorphisms (SNPs).

A fourth aspect of the present invention relates to a method of determining at least two genetic variations of nucleic acid sequences, comprising at least one genetic variation in a chromosome and at least one genetic variation in at least one plasmid, of a microorganism for a clinical isolate of the microorganism, particularly a bacterial microorganism, comprising:

obtaining or providing nucleic acid, e.g. gene, sequences of the clinical isolate of the microorganism, particularly the bacterial microorganism; and

determining the presence of the at least two genetic variations of the nucleic acid sequences comprising at least one genetic variation in a chromosome and at least one genetic variation in at least one plasmid in the nucleic acid, e.g. gene, sequences of the clinical isolate of the microorganism, particularly bacterial microorganism, as determined e.g. by the method of the first aspect.

With this method, antimicrobial drug, e.g. antibiotic, resistances in an unknown isolate of a microorganism, e.g. bacterial microorganism, can be determined.

A simple read out concept for a diagnostic test as described in this aspect can be as follows.

A sample, e.g. blood, from a patient, is used for molecular testing, e.g. using next generation sequencing (NGS), and then a molecular fingerprint is taken, e.g. in case of NGS a sequence of selected genomic/plasmid regions or the whole nucleic acid sequences, e.g. genome, is assembled. This is then compared to a reference library containing several reference sequences and/or a pan-genome, i.e. selected sequences or the whole sequence are/is compared to one or more reference sequences and/or a pan-genome, and structural variations (sequence / gene additions/deletions, etc.) and SNPs in the chromosomal nucleic acid sequences and the plasmid nucleic acid sequences are correlated with susceptibility/resistance profiles of reference sequences of the reference library. The reference library herein contains many nucleic acid sequences and/or one or more pan-genomes and is different from a reference genome. Then the result is reported, which can comprise ID (pathogen identification), i.e. a list of all (pathogenic) species identified in the sample, and AST (antimicrobial susceptibility testing), i.e. a list including a susceptibility /resistance profile for all species listed, based on genetic variations.

According to certain embodiments, statistical analysis in the present method is carried out using Fisher's test with $p < 10^{-3}$, preferably $p < 10^{-6}$, preferably $p < 10^{-9}$. Also, according to certain embodiments, the method further comprises correlating different genetic sites to each other.

Again, in the second, third and fourth aspect, the different steps herein can be carried out as described with regard to the first aspect of the present invention.

According to certain embodiments, the obtaining or providing of a sample containing or suspected of containing at least one microorganism, preferably a bacterial microorganism, e.g. one or more of Acinetobacter, Escherichia, e.g. E.coli, Enterobacter, Klebsiella, Proteus, Pseudomonas, Salmonella, Serratia, Shigella and/or Staphylococcus species, from the patient in the methods of the invention can comprise the following:

A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid sequences can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of the microorganism. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be analyzed as described with regard to the first to fourth aspect of the present invention.

In a fifth aspect the present invention relates to one or more computer program products comprising computer executable instructions which, when executed, perform a method according to any one of the first to the fourth aspect of the present invention.

In certain embodiments the computer program product is one on which program commands or program codes of a computer program for executing said method are stored. According to certain embodiments the computer program product is a storage medium. As noted above, the computer program products of the present invention can be self-learning, e.g. with respect to the first and second data sets.

In order to obtain the best possible information from the highly complex genetic data and develop an optimum model for diagnostic and therapeutical uses as well as the methods of the present invention - which can be applied stably in clinical routine - a thorough in silico analysis can be necessary. The proposed principle is based on a combination of different approaches, e.g. assembly of the nucleic acid, e.g. gene, sequences and/or genome of the microorganisms, at least in part and optionally annotating the sequences to one or more reference sequences and/or one or more pan-genomes, and/or alignment of the sequence data of the clinical isolate to be determined with one or more reference sequences and/or one or more pan-genomes, and correlation of structural variations and/or SNPs found in every sample in the chromosomal and plasmid nucleic acid sequence(s), e.g. from each patient, respectively an unknown clinical isolate, with all references and drugs, e.g. antibiotics, or only one or some of them, and search for structural variations and/or SNPs in the chromosomal and plasmid nucleic acid sequence(s) which occur for one or several drugs and in one or several strains.

Using the above steps a list of structural variations and/or SNPs in the chromosomal and plasmid nucleic acid sequence(s) with regard to one or more reference sequences and/or one or more pan-genomes is generated. This can be stored in databases, and statistical models can be derived from the databases. The statistical models can be based on at least one or more structural variations and/or at least one or more SNPs in the chromosomal and plasmid nucleic acid sequence(s). Statistical models that can be trained can be combined from structural variations, SNPs and/or sequences. Examples of algorithms that can produce such models are association Rules, Support Vector Machines, Decision Trees, Decision Forests, Discriminant-Analysis, Cluster-Methods, and many more.

The goal of the training is to allow a reproducible, standardized application during routine procedures.

For this, for example, nucleic acid, e.g. gene, sequences or parts thereof can be sequenced from a patient to be diagnosed. Afterwards, core characteristics can be derived from the sequence data which can be used to predict resistance. These are the points in the database used for the final model, i.e. at least one structural variation and/or one SNP in the chromosomal and plasmid nucleic acid sequence(s), but also combinations of one or more structural variations and/or one or more SNPs, etc.

The corresponding characteristics can be used as input for the statistical model and thus enable a prognosis for new patients. Not only the information regarding all resistances of all microorganisms, against all or only some or one drugs, e.g. antibiotics, can be integrated in a computer decision support tool, but also corresponding directives (e.g. EUCAST) so that only treatment proposals are made that are in line with the directives.

A sixth aspect of the present invention relates to the use of the computer program product according to the fifth aspect, e.g. for determining structural variations and/or SNPs in the chromosomal and plasmid nucleic acid sequence(s) of a microorganism for a clinical isolate of the microorganism in the fourth aspect of the invention and/or for use in the diagnostic method of the second method of the invention and/or for selecting a treatment in the third aspect of the present invention and/or in the method of the first aspect of the present invention.

A seventh aspect of the present invention is directed to a method of treating a patient suffering from an antimicrobial drug, e.g. antibiotic, resistant infection with a microorganism, preferably a bacterial microorganism, e.g. one or more of Acinetobacter, Escherichia, e.g. E.coli, Enterobacter, Klebsiella, Proteus, Pseudomonas, Salmonella, Serratia, Shigella and/or Staphylococcus species, comprising the steps of:

a) obtaining or providing a sample containing or suspected of containing at least one microorganism, preferably a bacterial microorganism, from the patient;
b) determining the presence of at least two genetic variations of the nucleic acid sequences comprising at least one genetic variation in the chromosome and at least one genetic variation in at least one plasmid, as determined by the method of the first aspect, wherein the presence of said at least two genetic variations of the nucleic acid sequences comprising at least one genetic variation in the chromosome and at least one genetic variation in at least one plasmid is indicative of a resistance to one or more antimicrobial drugs;
c) identifying said at least one or more antimicrobial, e.g. antibiotic, drugs;
d) selecting one or more antimicrobial, e.g. antibiotic, drugs different from the ones identified in step c) and being suitable for the treatment of the infection with the microorganism, preferably a bacterial microorganism; and
e) treating the patient with said one or more antimicrobial, e.g. antibiotic, drugs.

Herein, steps a) to d) can be carried out as described with respect to the fourth aspect. Step e) can be sufficiently carried out without being restricted and can be done e.g. non-invasively.

Examples

The present invention will now be described in detail with reference to several examples thereof. However, these examples are illustrative and do not limit the scope of the invention.

For analyzing the effect of considering the chromosomal and plasmid nucleic acids for determining antimicrobial resistance, six different bacterial strains of different genera were considered in the Examples to show that the effect is not limited to a particular strain or genus. Also, only SNPs were considered in the Examples shown below for easier analysis, although similar effects as shown below were also obtained for Examples wherein structural variants were taken into account.

The following strains/species were considered, and the number of individual samples in each case was as given in Table 1.

Table 1: Number of samples used for each species

| Species | Number of samples |
|---|---|
| *Acinetobacter baumannii* | 435 |
| *Escherichia coli* | 941 |
| *Klebsiella oxytoca* | 310 |
| *Klebsiella pneumoniae* | 1101 |
| *Proteus mirabilis* | 457 |
| *Serratia marcescens* | 510 |

Nucleic acid sequencing was carried out in addition to classical antimicrobial susceptibility testing of the same isolates. This allowed performing genome wide correlation studies to find genetic variants (e.g. point mutations, small insertions and deletion, larger structural variants, plasmid copy number gains, gene dosage effects) in the nucleic acid sequences that are significantly correlated to the resistance against one or several drugs. The approach also allows for comparing the relevant sites in the genome to each other.

For determining genetic variants on the plasmids, pan-genomes were constructed. For constructing the pan-genomes, the data obtained by sequencing of the samples, as detailed below, were assembled and calculated using Roary (Rapid large-scale prokaryote pan genome analysis (Bioinformatics 2015 Nov 15; 31 (22) : 3691-3. doi:

10.1093/bioinformatics/btv421. Epub 2015 Jul 20. Page AJ, Cummins CA, et al.). *De novo* assemblies were constructed using SPAdes (version 3.0.0, Bankevich A, Nurk S, Antipov D, et al. SPAdes: A New Genome Assembly Algorithm and Its Applications to Single-Cell Sequencing. Journal of Computational Biology. 2012;19(5):455-477. doi:10.1089/cmb.2012.0021) with parameters -t 20 -m 256 -k 21,33,55,77 --careful -1 fp.fastq.gz -2 rp.fastq.gz. To determine the quality of the assemblies we ran QUAST (version 2.3) with minimal length threshold of 500 bp. Resulting metric values not matching the RefSeq assembly quality criteria (N50 > 5000, L50 < 20, # contigs < 1000) were highlighted.

In the present approach the different sources of genetic resistance regarding structural variances as well as the different ways of how bacteria can become resistant were covered. By measuring clinical isolates collected in a broad geographical area and across a broad time span of three decades a complete picture going far beyond the rather artificial step of laboratory generated resistance mechanisms was tried to be generated.

To this end, a set of 21 clinically relevant antimicrobial agents with 5 different modes of action was put together, and the minimally inhibitory concentration (MIC) of the 21 drugs for the isolates was measured.

The detailed procedure is given in the following:

Bacterial Strains

The inventors selected strains from the microbiology strain collection at Siemens Healthcare Diagnostics (West Sacramento, CA) for susceptibility testing and nucleic acid sequencing.

Antimicrobial Susceptibility Testing (AST) Panels

Frozen reference AST panels were prepared following Clinical Laboratory Standards Institute (CLSI) recommendations. The following antimicrobial agents (with $\mu$g/ml concentrations shown in parentheses) were included in the panels: Amoxicillin/K Clavulanate (0.5/0.25-64/32), Ampicillin (0.25-128), Ampicillin/Sulbactam (0.5/0.25-64/32), Aztreonam (0.25-64), Cefazolin (0.5-32), Cefepime (0.25-64), Cefotaxime (0.25-128), Ceftazidime (0.25-64), Ceftriaxone (0.25-128), Cefuroxime (1-64), Cephalothin (1-64), Ciprofloxacin (0.015-8), Ertepenem (0.12-32), Gentamicin (0.12-32), Imipenem (0.25-32), Levofloxacin (0.25-16), Meropenem (0.12-32), Piperacillin/Tazobactam (0.25/4-256/4), Tetracycline (0.5-64), Tobramycin (0.12-32), and Trimethoprim/Sulfamethoxazole (0.25/4.7-32/608). Prior to use with clinical isolates, AST panels were tested with QC strains. AST panels were considered acceptable for testing with clinical isolates when the QC results met QC ranges described by CLSI16.

Inoculum Preparation

Isolates were cultured on trypticase soy agar with 5% sheep blood (BBL, Cockeysville, Md.) and incubated in ambient air at $35\pm1°C$ for 18-24 h. Isolated colonies (4-5 large colonies or 5-10 small colonies) were transferred to a 3 ml Sterile Inoculum Water (Siemens) and emulsified to a final turbidity of a 0.5 McFarland standard. 2 ml of this suspension was added to 25 ml Inoculum Water with Pluronic-F (Siemens). Using the Inoculator (Siemens) specific for frozen AST panels, 5 $\mu$l of the cell suspension was transferred to each well of the AST panel. The inoculated AST panels were incubated in ambient air at $35\pm1°C$ for 16-20 h. Panel results were read visually, and minimal inhibitory concentrations (MIC) were determined.

DNA extraction

Four streaks of each Gram-negative bacterial isolate cultured on trypticase soy agar containing 5% sheep blood and cell suspensions were made in sterile 1.5 ml collection tubes containing 50 $\mu$l Nuclease-Free Water (AM9930, Life Technologies). Bacterial isolate samples were stored at -20 °C until nucleic acid extraction. The Tissue Preparation System (TPS) (096D0382-02_01_B, Siemens) and the VERSANT® Tissue Preparation Reagents (TPR) kit (10632404B, Siemens) were used to extract DNA from these bacterial isolates. Prior to extraction, the bacterial isolates were thawed at room temperature and were pelleted at 2000 G for 5 seconds. The DNA extraction protocol DNAext was used for complete total nucleic acid extraction of 48 isolate samples and eluates, 50 $\mu$l each, in 4 hours. The total nucleic acid eluates were then transferred into 96-Well qPCR Detection Plates (401341, Agilent Technologies) for RNase A digestion, DNA quantitation, and plate DNA concentration standardization processes. RNase A (AM2271, Life Technologies) which was diluted in nuclease-free water following manufacturer's instructions was added to 50 $\mu$l of the total nucleic acid eluate for a final working concentration of 20 $\mu$g/ml. Digestion enzyme and eluate mixture were incubated at 37°C for 30 minutes using Siemens VERSANT® Amplification and Detection instrument. DNA from the RNase digested eluate was quantitated using the Quant-iT™ PicoGreen dsDNA Assay (P11496, Life Technologies) following the assay kit instruction, and fluorescence was determined on the Siemens VERSANT® Amplification and Detection instrument. Data analysis was performed using Microsoft® Excel 2007. 25 $\mu$l of the quantitated DNA eluates were transferred into a new 96-well PCR plate for plate DNA concentration standardization prior to library preparation. Elution buffer from the TPR kit was used to adjust DNA concentration. The standardized DNA eluate plate was then stored at -80°C until library preparation.

Next Generation Sequencing

Prior to library preparation, quality control of isolated bacterial DNA was conducted using a Qubit 2.0 Fluorometer (Qubit dsDNA BR Assay Kit, Life Technologies) and an Agilent 2200 TapeStation (Genomic DNA ScreenTape, Agilent Technologies). NGS libraries were prepared in 96 well format using NexteraXT DNA Sample Preparation Kit and NexteraXT Index Kit for 96 Indexes (Illumina) according to the manufacturer's protocol. The resulting sequencing libraries were quantified in a qPCR-based approach using the KAPA SYBR FAST qPCR MasterMix Kit (Peqlab) on a ViiA 7 real time PCR system (Life Technologies). 96 samples were pooled per lane for paired-end sequencing (2x 100bp) on Illumina Hiseq2000 or Hiseq2500 sequencers using TruSeq PE Cluster v3 and TruSeq SBS v3 sequencing chemistry (Illumina). Basic sequencing quality parameters were determined using the FastQC quality control tool for high throughput sequence data (Babraham Bioinformatics Institute).

Afterwards, the data were mapped and analyzed differently for a model that takes into account only chromosomal nucleic acid sequences, hereinafter also called chromosomal model, and for a model that takes into account both chromosomal and plasmid nucleic acid sequences, hereinafter also called PG model.

**Mapping:**

Mapping was carried out for both models against the pan-genomes given above and in the accompanying sequence protocol.

For the chromosomal model, mapping was furthermore also carried out against specific reference genomes that take into account only nucleic acid sequences on the chromosome, which are shown in the accompanying sequence protocol and are as follows: SEQ ID NO 1 showing the reference genome (NC_017847 as annotated at the NCBI) for *Acinetobacter baumannii,* SEQ ID NO 2 showing the reference genome (CP000948, as annotated at the NCBI) for *Escherichia coli,* SEQ ID NO 3 showing the reference genome (NC_009648, as annotated at the NCBI) for *Klebsiella pneumoniae,* SEQ ID NO 4 showing the reference genome (NC_016612, as annotated at the NCBI) for *Klebsiella oxytoca,* SEQ ID NO 5 showing the reference genome (NC_010554 as annotated at the NCBI) for *Proteus mirabilis,* and SEQ ID NO 6 showing the reference genome (NC_020211 as annotated at the NCBI) for *Serratia marcescens.*

The reference genomes were thereby selected for each bacterial species by correlating the data to all chromosomal reference genomes used in the pan-genome and selecting the best suited.

Reference sequence for *Acinetobacter baumannii,* strain NC_017847 (http://www.ncbi.nlm.nih.gov/nuccore/NC_017847) LOCUS NC_017847 3964912 bp DNA circular CON 01-MAR-2015 DEFINITION Acinetobacter baumannii MDR-TJ, complete genome. ACCESSION NC_017847 NZ_AEOE01000000 NZ_AEOE01000001 NZ_AEOE01000002
NZ_AEOE01000003 NZ_AEOE01000004
VERSION NC_017847.1 GI:387122089
DBLINK BioProject: PRJNA224116
BioSample: SAMN02603104
Assembly: GCF_000187205.2
KEYWORDS RefSeq.
SOURCE Acinetobacter baumannii MDR-TJ
ORGANISM Acinetobacter baumannii MDR-TJ
Bacteria; Proteobacteria; Gammaproteobacteria; Pseudomonadales; Moraxellaceae; Acinetobacter; Acinetobacter calcoaceticus/baumannii complex.

REFERENCE 1 (bases 1 to 3964912)
AUTHORS Huang,H., Yang,Z.L., Wu,X.M., Wang,Y., Liu,Y.J., Luo,H., Lv,X., Gan,Y.R., Song,S.D. and Gao,F. TITLE Complete genome sequence of Acinetobacter baumannii MDR-TJ and insights into its mechanism of antibiotic resistance JOURNAL J. Antimicrob. Chemother. 67 (12), 2825-2832 (2012)
PUBMED 22952140
REFERENCE 2 (bases 1 to 3964912)
AUTHORS Gao,F., Wang,Y., Liu,Y.J., Wu,X.M., Lv,X., Gan,Y.R., Song,S.D. and Huang,H. TITLE Genome sequence of Acinetobacter baumannii MDR-TJ JOURNAL J. Bacteriol. 193 (9), 2365-2366 (2011)
PUBMED 21398552
REFERENCE 3 (bases 1 to 3964912)
AUTHORS Huang,H., Yang,Z.-L., Wu,X.-M., Wang,Y., Liu,Y.-J., Luo,H., Lv,X., Gan,Y.-R., Song,S.-D. and Gao,F. TITLE Direct Submission JOURNAL Submitted (06-APR-2012) Department of Physics, Tianjin University, No.92, Weijin Road, Nankai District, Tianjin 300072, China

Reference sequence for *Escherichia coli,* str. K-12 substr. DH10B:

LOCUS CP000948 4686137 bp DNA circular BCT 05-JUN-2008
DEFINITION Escherichia coli str. K12 substr. DH10B, complete genome.

ACCESSION CP000948

VERSION CP000948.1 GI:169887498
DBLINK BioProject: PRJNA20079

KEYWORDS

SOURCE Escherichia coli str. K-12 substr. DH10B
ORGANISM Escherichia coli str. K-12 substr. DH10B Bacteria; Proteobacteria; Gammaproteobacteria; Enterobacteriales; Enterobacteriaceae; Escherichia.

REFERENCE 1 (bases 1 to 4686137)
AUTHORS Durfee,T., Nelson,R., Baldwin,S., Plunkett,G. III, Burland,V., Mau,B., Petrosino,J.F., Qin,X., Muzny,D.M.,

Ayele,M., Gibbs,R.A., Csorgo,B., Posfai,G., The inventorsinstock,G.M. and Blattner,F.R. TITLE The complete genome sequence of Escherichia coli DH10B: insights into the biology of a laboratory workhorse JOURNAL J. Bacteriol. 190 (7), 2597-2606 (2008)
PUBMED 18245285
REFERENCE 2 (bases 1 to 4686137)
AUTHORS Plunkett,G. III. TITLE Direct Submission JOURNAL Submitted (20-FEB-2008) Department of Genetics and Biotechnology, University of Wisconsin, 425G Henry Mall, Madison, WI 53706, USA
COMMENT DH10B and DH10B-T1R are available from Invitrogen Corporation (http://www.invitrogen.com).

Reference sequence for *Klebsiella oxytoca,* strain NC_016612 (http://www.genome.jp/dbget-bin/www_bget?refseq+NC_016612) LOCUS NC_0166125974109 bp DNA circular CON 07-FEB-2015 DEFINITION Klebsiella oxytoca KCTC 1686, complete genome.

ACCESSION NC_016612

VERSION NC_016612.1 GI:375256816 DBLINK BioProject: PRJNA224116 BioSample: SAMN02603580 Assembly: GCF_000240325.1
KEYWORDS RefSeq.
SOURCE Klebsiella oxytoca KCTC 1686
ORGANISM Klebsiella oxytoca KCTC 1686 Bacteria; Proteobacteria; Gammaproteobacteria; Enterobacteriales; Enterobacteriaceae; Klebsiella.

REFERENCE 1 (bases 1 to 5974109)
AUTHORS Shin,S.H., Kim,S., Kim,J.Y., Lee,S., Um,Y., Oh,M.K., Kim,Y.R., Lee,J. and Yang,K.S. TITLE Complete genome sequence of Klebsiella oxytoca KCTC 1686, used in production of 2,3-butanediol JOURNAL J. Bacteriol. 194 (9), 2371-2372 (2012)
PUBMED 22493189
REFERENCE 2 (bases 1 to 5974109)
AUTHORS Shin,S.H., Kim,S., Kim,J.Y., Yang,K.-S. and Seo,J.-S. TITLE Direct Submission JOURNAL Submitted (21-DEC-2011) Life Science Institute, Macrogen Inc., 10F, World Meridian Center, 60-24, Gasan-dong, Kumchun-gu, Seoul 153-781, Republic of Korea

Reference sequence for *Klebsiella pneumoniae,* strain NC_009648 (http://www.genome.jp/dbget-bin/www_bget?refseq+NC_009648)
LOCUS NC_009648 5315120 bp DNA circular CON 07-FEB-2015 DEFINITION Klebsiella pneumoniae subsp. pneumoniae MGH 78578, complete sequence.

ACCESSION NC_009648

VERSION NC_009648.1 GI:152968582 DBLINK BioProject: PRJNA224116 BioSample: SAMN02603941 Assembly: GCF_000016305.1
KEYWORDS RefSeq.
SOURCE Klebsiella pneumoniae subsp. pneumoniae MGH 78578
ORGANISM Klebsiella pneumoniae subsp. pneumoniae MGH 78578
Bacteria; Proteobacteria; Gammaproteobacteria; Enterobacteriales; Enterobacteriaceae; Klebsiella.

REFERENCE 1 (bases 1 to 5315120)
AUTHORS McClelland,M., Sanderson,E.K., Spieth,J., Clifton,W.S., Latreille,P., Sabo,A., Pepin,K., Bhonagiri,V., Porwollik,S., Ali,J. and Wilson,R.K. CONSRTM The Klebsiella pneumonia Genome Sequencing Project TITLE Direct Submission JOURNAL Submitted (06-SEP-2006) Genetics, Genome Sequencing Center, 4444 Forest Park Parkway, St. Louis, MO 63108, USA
Reference sequence for *Proteus mirabilis,* strain NC_010554 (http://www.genome.jp/dbget-bin/www_bget?refseq+NC_010554) LOCUS NC_010554 4063606 bp DNA circular CON 07-FEB-2015 DEFINITION Proteus mirabilis strain HI4320, complete genome.

ACCESSION NC_010554

VERSION NC_010554.1 GI:197283915
DBLINK BioProject: PRJNA224116
Assembly: GCF_000069965.1
KEYWORDS RefSeq; complete genome.
SOURCE Proteus mirabilis HI4320
ORGANISM Proteus mirabilis HI4320 Bacteria; Proteobacteria; Gammaproteobacteria; Enterobacteriales; Enterobacteriaceae; Proteus.

REFERENCE 1
AUTHORS Pearson,M.M., Sebaihia,M., Churcher,C., Quail,M.A., Seshasayee,A.S., Luscombe,N.M., Abdellah,Z., Arrosmith,C., Atkin,B., Chillingworth,T., Hauser,H., Jagels,K., Moule,S., Mungall,K., Norbertczak,H., Rabbinowitsch,E., Walker,D., Whithead,S., Thomson,N.R., Rather,P.N., Parkhill,J. and Mobley,H.L. TITLE Complete genome sequence of uropathogenic Proteus mirabilis, a master of both adherence and motility JOURNAL J. Bacteriol. 190 (11), 4027-4037 (2008)
PUBMED 18375554
REFERENCE 2 (bases 1 to 4063606)
AUTHORS Sebaihia,M. TITLE Direct Submission JOURNAL Submitted (18-FEB-2008) Sebaihia M., Sulston Laboratories, Wellcome Trust Sanger Institute, Wellcome Trust Genome Campus, Hinxton, Cambridge, CB10 1SA, UNITED KINGDOM

Reference sequence for *Serratia marcescens,* strain NC_020211 (http://www.genome.jp/dbget-bin/www_bget?refseq+NC_020211) LOCUS NC_020211 5241455 bp DNA circular CON 07-FEB-2015 DEFINITION Serratia marcescens WW4, complete genome.

ACCESSION NC_020211

VERSION NC_020211.1 GI:448239774
DBLINK BioProject: PRJNA224116 BioSample: SAMN02602965 Assembly: GCF_000336425.1
KEYWORDS RefSeq. SOURCE Serratia marcescens WW4
ORGANISM Serratia marcescens WW4 Bacteria; Proteobacteria; Gammaproteobacteria; Enterobacteriales; Enterobacteriaceae; Serratia.

REFERENCE 1 (bases 1 to 5241455)
AUTHORS Kuo,P.A., Kuo,C.H., Lai,Y.K., Graumann,P.L. and Tu,J. TITLE Phosphate limitation induces the intergeneric inhibition of Pseudomonas aeruginosa by Serratia marcescens isolated from paper machines JOURNAL FEMS Microbiol. Ecol. 84 (3), 577-587 (2013) PUBMED 23398522
REFERENCE 2 (bases 1 to 5241455)
AUTHORS Chung,W.C., Chen,L.L., Lo,W.S., Kuo,P.A., Tu,J. and Kuo,C.H. TITLE Complete Genome Sequence of Serratia marcescens WW4 JOURNAL Genome Announc 1 (2), E0012613 (2013)
PUBMED 23558532
REMARK Publication Status: Online-Only
REFERENCE 3 (bases 1 to 5241455)
AUTHORS Chung,W.-C., Chen,L.-L., Lo,W.-S., Kuo,P.-A., Tu,J. and Kuo,C.-H. TITLE Direct Submission JOURNAL Submitted (26-NOV-2012) Institute of Plant and Microbial Biology, Academia Sinica, 128 Sec. 2, Academia Rd., Taipei 115, Taiwan

Raw paired-end sequencing data for the samples were mapped against the respective pan-genomes with BWA 0.6.1.20. The resulting SAM files were sorted, converted to BAM files, and PCR duplicates were marked using the Picard tools package 1.104 (http://picard.sourceforge.net/).

**Data analysis**

For the chromosomal model, analysis was as follows:

The Genome Analysis Toolkit 3.1.1 (GATK) was used to call SNPs and indels for blocks of 200 samples (parameters: - ploidy 1 -glm BOTH -stand_call_conf 30 -stand_emit_conf 10). VCF files were combined into a single file and quality filtering for SNPs was carried out (QD < 2.0 ‖ FS > 60.0 ‖ MQ < 40.0) and indels (QD < 2.0‖ FS > 200.0). Detected variants were annotated with SnpEff22 to predict coding effects.

For matching the obtained SNPs in the reference genomes with the pan-genomes, the following analysis was carried out:

1. The gene containing the variant was identified (gene entry in the corresponding GenBank file of the reference genome), i.e. the genomic position of the variant must be within the gene start and end interval. The start and end positions of the subsequence containing the variant were determined as follows: Start was set to (genomic positon of the variant - 250 bases) and end to (genomic position of the variant + 250 bases). If the start/end position were beyond the gene start/end then they were set to start/end positions of the gene.
2. The extracted variant containing subsequences were aligned against the pan-genome of the corresponding species using blastn.
3. The blastn results were filtered: Only matches with sequence identity >= 80% and aligning >= 80% of the variant containing subsequence were kept.
4. For each of the remaining matches the position of the variant within the pan-genome gene was determined.
5. These positions were searched in the corresponding filtered VCF files (i.e. VCF (Variant Call Format) file containing analyzed variants). For easier analysis, only the 50 SNPs with the best p-values in the SNP analysis were taken into account. Only variants which could be mapped to the pan-genome and were found in corresponding VCF files were considered: They were coded as binary variables: 0 = no reference allele, 1 = reference allele, NA = missing.

Resistance profiles were determined with w.r.t. EUCAST MIC breakpoint guidelines (v. 4). Samples considered were only samples after filtering w.r.t. assembly quality and taxonomic assignment. For obtaining an optimized model, decision tree analysis was carried out as follows:

Model: Decision tree from R-package rpart; maximal depth=5, other parameters set to "force" more complex trees containing multiple features; the set parameters affect the number of features in the model

For the PG model, the analysis was as follows:

10 repetitions of 5-fold cross-validation were carried out. In each fold, features were selected by ranking them using a GWAS (genome-wide association study) approach with PCA (principal component analysis) adjustment. The final model was built from the most occurring features from the cross-validation. For obtaining an optimized model, decision tree analysis was carried out as follows:

Model: Decision tree from R-package rpart; maximal depth=5, default parameters, pruning (decreasing tree size to avoid over fitting; the set parameters affect the number of features in the model

Due to the different approaches taken for the analysis of only the chromosomal nucleic acid sequence on the one hand and for the analysis of both chromosomal and plasmid nucleic acid sequences on the other hand, a comparison of the data sets was actually quite difficult in some cases, so that only selected cases are shown in the following.
Selected results for the different species are given in tables 2 to 7. In the tables, the column "drug" refers to the respective antibiotic used in each exemplary analysis, columns 2 and 3 refer to the model using only chromosomal nucleic acid sequences, and columns 4 and 5 refers to the model that takes into account both chromosomal and plasmid nucleic acid sequences (termed "PG model"). Further, the term "B_ACC" refers to the balanced accuracy, and the term "features" refers to the different SNPs that were used in the decision trees obtained for the respective models in tables 2 to 7.
The balanced accuracy was thereby used for analyzing the data as it gives more balanced results, as explained in the following.

```
The balanced accuracy is defined as the arithmetic mean of
sensitivity and specificity = (sensitivity + specificity)/2
with sensitivity = TP / (TP+FN) and specificity = TN /
(TN+FP).
```

• TN = true negatives = susceptible and predicted to be susceptible

- TP = true positives = resistant and predicted to be resistant
- FN = false negatives = resistance, predicted to be susceptible
- FP = false positives = susceptible, predicted to be resistance

It is a better performance estimate than accuracy ( (TP + TN) / (number of samples)) in case of imbalanced datasets, e.g. if there are much more resistant samples when non-resistant ones or vice versa. In such cases accuracy may be high, although the "smaller" class is not predicted correctly, as seen in the following exemplary numerical case - the balanced accuracy is less biased by the data imbalance.

Exemplary numerical case: 11 samples are resistant, 51 are susceptible and TP=50, TN=1, FN=1, FP=10. Then accuracy = (50+1) / 62 = 82.26% and balanced accuracy is (( 50/51) + ( 1/11 ))/2 = 53.57%.

A comparison of the two models is only shown in the subsequent tables if both models have at least 2 unique variants.

Table 2: Results of Examples for *Acinetobacter baumannii*

| Drug | B_ACC using only chromosome | number of features (chromosome) | B_ACC (PG model) | PG model number of features |
|------|------|------|------|------|
| CP | 80.7 | 4 | 85.34 | 7 |
| IMP | 61.2 | 3 | 62.11 | 2 |
| LVX | 89.35 | 7 | 91.48 | 3 |

Table 3: Results of Examples for *Escherichia coli*

| Drug | B_ACC using only chromosome | number of features (chromosome) | B_ACC (PG model) | PG model number of features |
|------|------|------|------|------|
| CRM | 55.65 | 8 | 62.71 | 7 |

Table 4: Results of Examples for *Klebsiella oxytoca*

| Drug | B_ACC using only chromosome | number of features (chromosome) | B_ACC (PG model) | PG model number of features |
|------|------|------|------|------|
| CP | 93.05 | 3 | 93.76 | 2 |

Table 5: Results of Examples for *Klebsiella pneumoniae*

| Drug | B_ACC using only chromosome | number of features (chromosome) | B_ACC (PG model) | PG model number of features |
|------|------|------|------|------|
| A/S | 72.6 | 5 | 77.48 | 4 |
| AZT | 88.5 | 6 | 88.81 | 2 |
| CAZ | 87.65 | 6 | 87.98 | 4 |
| CRM | 79.65 | 7 | 81.83 | 4 |
| GM | 70.45 | 8 | 70.47 | 12 |

Table 6: Results of Examples for *Proteus mirabilis*

| Drug | B_ACC using only chromosome | number of features (chromosome) | B_ACC (PG model) | PG model number of features |
|------|------|------|------|------|
| AM | 70.75 | 2 | 74.46 | 4 |
| A/S | 51.3 | 2 | 62.91 | 2 |
| CP | 86.35 | 2 | 89.11 | 5 |
| LVX | 83.75 | 2 | 87.77 | 4 |
| T/S | 68.45 | 2 | 71.35 | 3 |

Table 7: Results of Examples for *Serratia marcescens*

| Drug | B_ACC using only chromosome | number of features (chromosome) | B_ACC (PG model) | PG model number of features |
|------|------|------|------|------|
| AZT | 58.7 | 8 | 69.67 | 5 |
| CAX | 58.1 | 5 | 68.08 | 2 |
| CAZ | 60.05 | 3 | 62.82 | 5 |
| CFT | 58.6 | 6 | 67.45 | 5 |

As can be seen from the tables, the balanced accuracy - and therefore the prediction of antibiotic resistance - improved for the different tested drugs all over the different bacterial species using the nucleic acid information from both the chromosome and the plasmids.

It is noted that the number of features in the tables for achieving the best results in balanced accuracy often vary, which is a result of the optimization approach which was used in the present models for achieving these results and which were not limited to achieve certain comparable numbers of features in the decision trees. Notably, though, the variances in numbers of features shifts in both directions, i.e. sometimes more feature numbers are obtained in the model using chromosomal nucleic acid sequences only, and sometimes more feature numbers are obtained in the PG model, showing that there is no bias in any of the models for a higher number of features. Further, the data for the drug A/S for Proteus mirabilis actually had the same number of features for both models, which show that the effect is also independent of the number of features and can be obtained for a combination of at least two features. It is to be noted that the features in the two models in this case also differed. Overall an improvement in performance is seen when the plasmid nucleic acid sequences are taken into account.

Further to the results in the tables, it was also again confirmed that a combination of more than one, e.g. i (being a natural number), variants performed significantly better than single variants. It was also seen in the data that the power of predicting genetic resistance increased if variants from different genes were selected.

While in the first step all i variants could have been theoretically selected from the same gene, the performance increased when j (again being a natural number) variants are selected that come from at least two different genes. Further improved results were obtained when the mutations were selected from the chromosome and the plasmid, as seen in the above tables.

In the examples, it was demonstrated that the performance of predicting resistance of the bacteria further increases if the full set of all genes from the chromosome and the plasmid is used. By using the full genetic set available, we outper-formed the results that are solely based on the bacterial chromosome, demonstrating that adding the plasmid information and combining it with the chromosomal genes is an important step towards improved prediction of bacterial resistance.

As already stated above, notably the information described herein does not only refer to single variants (SNPs) and combinations of those. The same also applies for the above-mentioned larger structural variations; also in this case the performance gets better if genes from bacterial chromosomes and plasmids are included in the bioinformatics analysis as compared to the performance of single genes from the chromosome or combinations of genes from the chromosomes of bacteria.

**Claims**

1. A method of determining an antimicrobial drug resistance profile for a microorganism, comprising:

   - obtaining or providing a first data set of nucleic acid sequences of a plurality of clinical isolates of the microorganism, wherein at least a part of the nucleic acid sequences of the first data set are assembled; and/or obtaining or providing a first data set of nucleic acid sequences of a plurality of clinical isolates of the microorganism and aligning the nucleic acid sequences of the first data set to at least one reference sequence;
   - analyzing the nucleic acid sequences of the first data set for at least two genetic variations of the nucleic acid sequences comprising at least one genetic variation in a chromosome and at least one genetic variation in at least one plasmid to obtain a third data set of structural variants;
   - providing a second data set of antimicrobial drug resistance and/or susceptibility of the plurality of clinical isolates of the microorganism;
   - correlating the third data set with the second data set and statistically analyzing the correlation; and
   - determining the genetic variations in the nucleic acid sequences of the microorganism associated with antimicrobial drug resistance.

**2.** The method of claim 1, wherein the genetic variations are annotated to a pan-genome of the microorganism and/or annotated to one or more reference genomes.

**3.** The method of one or more of the preceding claims, wherein the method involves determining the resistance of the microorganism to one or more antimicrobial drugs.

**4.** The method of one or more of the preceding claims, wherein the antimicrobial drug is selected from the group consisting of Amoxicillin/K Clavulanate (AUG), Ampicillin (AM), Aztreonam (AZT), Cefazolin (CFZ), Cefepime (CPE), Cefotaxime (CFT), Ceftazidime (CAZ), Ceftriaxone (CAX), Ce-furoxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T), Ampicillin/Sulbactam (A/S), Tetracycline (TE), Tobramycin (TO), and Trimethoprim/Sulfamethoxazole (T/S).

**5.** The method of one or more of the preceding claims, wherein the resistance of the microorganism against 1, 2, 3, 4, 5, 6, 7, 8, 9, 10, 11, 12, 13, 14, 15 or 16, 17, 18, 19, 20 or 21 antibiotic drugs is determined.

**6.** A method of determining an infection of a patient with an antimicrobial drug resistant microorganism, comprising the steps of:

a) obtaining or providing a sample containing or suspected of containing a microorganism from the patient;
b) determining the presence of at least two genetic variations of the nucleic acid sequences comprising at least one genetic variation in the chromosome and at least one genetic variation in at least one plasmid, as determined by the method of any one of claims 1 to 5, wherein the presence of said at least two genetic variations of the nucleic acid sequences comprising at least one genetic variation in the chromosome and at least one genetic variation in at least one plasmid is indicative of an infection with an antimicrobial drug resistant microorganism in said patient.

**7.** The method of claim 6, wherein the microorganism is a bacterial microorganism potentially resistant to antimicrobial drug treatment.

**8.** A method of selecting a treatment of a patient suffering from an infection with a potentially antimicrobial drug resistant microorganism, comprising the steps of:

a) obtaining or providing a sample containing or suspected of containing a microorganism from the patient;
b) determining the presence of at least two genetic variations of the nucleic acid sequences comprising at least one genetic variation in the chromosome and at least one genetic variation in at least one plasmid, as determined by the method of any one of claims 1 to 5, wherein the presence of said at least two genetic variations of the nucleic acid sequences comprising at least one genetic variation in the chromosome and at least one genetic variation in at least one plasmid is indicative of a resistance to one or more antimicrobial drugs;
c) identifying said at least one or more antimicrobial drugs; and
d) selecting one or more antimicrobial drugs different from the ones identified in step c) and being suitable for the treatment of the infection with the microorganism.

**9.** The method of one or more of claims 1 to 8, wherein the genetic variation is selected from at least one of structural variations of the nucleic acid sequences comprising at least a change in the nucleic acid sequence comprising more than one base, and single nucleotide polymorphisms (SNPs).

**10.** The method of one or more of claims 6 to 9, wherein determining the nucleic acid sequence information or the presence of a genetic variation comprises using a next generation sequencing or high throughput sequencing method.

**11.** Computer program product comprising computer executable instructions which, when executed, perform a method according to any one of claims 1 to 10.

Europäisches
Patentamt

European
Patent Office

Office européen
des brevets

# EUROPEAN SEARCH REPORT

Application Number

EP 16 16 5319

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | N. STOESSER ET AL: "Predicting antimicrobial susceptibilities for Escherichia coli and Klebsiella pneumoniae isolates using whole genomic sequence data", JOURNAL OF ANTIMICROBIAL CHEMOTHERAPY, vol. 4, 30 May 2013 (2013-05-30), XP055157002, ISSN: 0305-7453, DOI: 10.1093/jac/dkt180 * the whole document * | 1-11 | INV. C12Q1/68 |
| X | NICOLE D PECORA ET AL: "Genomically Informed Surveillance for Carbapenem-Resistant Enterobacteriaceae in a Health Care System", MBIO, AMERICAN SOCIETY FOR MICROBIOLOGY, US, vol. 6, no. 4, 28 July 2015 (2015-07-28), pages e01030-1, XP002758126, ISSN: 2161-2129, DOI: 10.1128/MBIO.01030-15 * the whole document * | 1-11 | |
| X | N. C. GORDON ET AL: "Prediction of Staphylococcus aureus Antimicrobial Resistance by Whole-Genome Sequencing", JOURNAL OF CLINICAL MICROBIOLOGY, vol. 52, no. 4, 1 April 2014 (2014-04-01), pages 1182-1191, XP055280452, US ISSN: 0095-1137, DOI: 10.1128/JCM.03117-13 * the whole document * | 1-11 | TECHNICAL FIELDS SEARCHED (IPC)  C12Q |

-/--

The present search report has been drawn up for all claims

| Place of search | Date of completion of the search | Examiner |
|---|---|---|
| Munich | 11 October 2016 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 16 16 5319

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | S. ZHAO ET AL: "Whole-Genome Sequencing Analysis Accurately Predicts Antimicrobial Resistance Phenotypes in Campylobacter spp.", APPLIED AND ENVIRONMENTAL MICROBIOLOGY, vol. 82, no. 2, 15 January 2016 (2016-01-15), pages 459-466, XP055309391, US ISSN: 0099-2240, DOI: 10.1128/AEM.02873-15 * the whole document * | 1-5 | |
| X | US 2012/071336 A1 (BALASHOV SERGEY [US] ET AL) 22 March 2012 (2012-03-22) * claims 1-16; examples 1,7,8 * | 6-11 | |
| A | PRIYANKA BAJAJ ET AL: "Escherichia coli [beta]-Lactamases: What Really Matters", FRONTIERS IN MICROBIOLOGY, vol. 7, 30 March 2016 (2016-03-30), page 417, XP055309492, DOI: 10.3389/fmicb.2016.00417 * the whole document * | 1-11 | |

| | | | TECHNICAL FIELDS SEARCHED (IPC) |
|---|---|---|---|

The present search report has been drawn up for all claims

| Place of search | Date of completion of the search | Examiner |
|---|---|---|
| Munich | 11 October 2016 | Bradbrook, Derek |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 16 16 5319

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2016

| Patent document cited in search report | Publication date | Patent family member(s) | Publication date |
|---|---|---|---|
| US 2012071336 A1 | 22-03-2012 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WOZNIAK et al.** *BMC Genomics,* 2012, vol. 13 (7), 23
- *J Antimicrob Chemother,* 2013, vol. 68, 2234-2244
- **CHEWAPREECHA et al.** Comprehensive Identification of single nucleotide polymorphisms associated with beta-lactam resistance within pneumococcal mosaic genes. *PLoS Genet,* 2014, vol. 10 (8), e1004547
- **FORTH ; HENSCHLER ; RUMMEL.** Allgemeine und spezielle Pharmakologie und Toxikologie. 2005, 781-919
- Remington, The Science and Practice of Pharmacy. 2013, 777-1070
- **CUMMINS CA.** *Bioinformatics,* 15 November 2015, vol. 31 (22), 3691-3
- **BANKEVICH A ; NURK S ; ANTIPOV D et al.** SPAdes: A New Genome Assembly Algorithm and Its Applications to Single-Cell Sequencing. *Journal of Computational Biology,* 2012, vol. 19 (5), 455-477
- **AUTHORS HUANG,H. ; YANG,Z.L. ; WU,X.M. ; WANG,Y. ; LIU,Y.J. ; LUO,H. ; LV,X. ; GAN,Y.R. ; SONG,S.D. ; GAO,F.** TITLE Complete genome sequence of Acinetobacter baumannii MDR-TJ and insights into its mechanism of antibiotic resistance. *JOURNAL J. Antimicrob. Chemother.,* 2012, vol. 67 (12), 2825-2832
- **AUTHORS GAO,F. ; WANG,Y. ; LIU,Y.J. ; WU,X.M. ; LV,X. ; GAN,Y.R. ; SONG,S.D. ; HUANG,H.** TITLE Genome sequence of Acinetobacter baumannii MDR-TJ. *JOURNAL J. Bacteriol.,* 2011, vol. 193 (9), 2365-2366
- TITLE Direct Submission. **AUTHORS HUANG,H. ; YANG,Z.-L. ; WU,X.-M. ; WANG,Y. ; LIU,Y.-J. ; LUO,H. ; LV,X. ; GAN,Y.-R. ; SONG,S.-D. ; GAO,F.** JOURNAL Submitted (06-APR-2012) Department of Physics. Tianjin University
- **AUTHORS DURFEE,T. ; NELSON,R. ; BALDWIN,S. ; PLUNKETT,G. III ; BURLAND,V. ; MAU,B. ; PETROSINO,J.F. ; QIN,X. ; MUZNY,D.M. ; AYELE,M.** TITLE The complete genome sequence of Escherichia coli DH10B: insights into the biology of a laboratory workhorse. *JOURNAL J. Bacteriol.,* 2008, vol. 190 (7), 2597-2606
- TITLE Direct Submission. **AUTHORS PLUNKETT,G. III.** JOURNAL Submitted (20-FEB-2008) Department of Genetics and Biotechnology. University of Wisconsin

- **AUTHORS SHIN,S.H. ; KIM,S. ; KIM,J.Y. ; LEE,S. ; UM,Y. ; OH,M.K. ; KIM,Y.R. ; LEE,J. ; YANG,K.S.** TITLE Complete genome sequence of Klebsiella oxytoca KCTC 1686, used in production of 2,3-butanedio. *JOURNAL J. Bacteriol.,* 2012, vol. 194 (9), 2371-2372
- TITLE Direct Submission. **AUTHORS SHIN,S.H. ; KIM,S. ; KIM,J.Y. ; YANG,K.-S. ; SEO,J.-S.** JOURNAL Submitted. Life Science Institute, Macrogen Inc, 21 December 2011
- CONSRTM The Klebsiella pneumonia Genome Sequencing Project TITLE Direct Submission. **AUTHORS MCCLELLAND,M. ; SANDERSON,E.K. ; SPIETH,J. ; CLIFTON,W.S. ; LATREILLE,P. ; SABO,A. ; PEPIN,K. ; BHONAGIRI,V. ; PORWOLLIK,S. ; ALI,J.** JOURNAL Submitted. Genetics, Genome Sequencing Center, 06 September 2006
- **AUTHORS PEARSON,M.M. ; SEBAIHIA,M. ; CHURCHER,C. ; QUAIL,M.A. ; SESHASAYEE,A.S. ; LUSCOMBE,N.M. ; ABDELLAH,Z. ; ARROSMITH,C. ; ATKIN,B. ; CHILLINGWORTH,T.** TITLE Complete genome sequence of uropathogenic Proteus mirabilis, a master of both adherence and motility. *JOURNAL J. Bacteriol.,* 2008, vol. 190 (11), 4027-4037
- TITLE Direct Submission. **AUTHORS SEBAIHIA,M.** JOURNAL Submitted. Wellcome Trust Sanger Institute, 18 February 2008
- **AUTHORS KUO,P.A. ; KUO,C.H. ; LAI,Y.K. ; GRAUMANN,P.L. ; TU,J.** TITLE Phosphate limitation induces the intergeneric inhibition of Pseudomonas aeruginosa by Serratia marcescens isolated from paper machines. *JOURNAL FEMS Microbiol. Ecol.,* 2013, vol. 84 (3), 577-587
- **AUTHORS CHUNG,W.C. ; CHEN,L.L. ; LO,W.S. ; KUO,P.A. ; TU,J. ; KUO,C.H.** TITLE Complete Genome Sequence of Serratia marcescens WW4. *JOURNAL Genome Announc,* 2013, vol. 1 (2), E0012613
- TITLE Direct Submission. **AUTHORS CHUNG,W.-C. ; CHEN,L.-L. ; LO,W.-S. ; KUO,P.-A. ; TU,J. ; KUO,C.-H.** JOURNAL Submitted. Institute of Plant and Microbial Biology, 26 November 2012